# EUROPÄISCHE PATENTSCHRIFT

(11) **EP 0 906 260 B1**
(45) Veröffentlichungstag und Bekanntmachung des Hinweises auf die Patenterteilung: **03.01.2001**
(21) Anmeldenummer: 97929163.0
(22) Anmeldetag: 11.06.1997
(51) Int. Cl.: C07C 45/46, C07C 49/675

(54) **VERFAHREN ZUR HERSTELLUNG SUBSTITUIERTER INDANONE**
PROCESS FOR PRODUCING SUBSTITUTED INDANONES
PROCEDE DE PRODUCTION D'INDANONES SUBSTITUEES

(30) Priorität: 21.06.1996 DE 19624828
(43) Veröffentlichungstag der Anmeldung: 07.04.1999
(73) Patentinhaber: BASF AKTIENGESELLSCHAFT, 67056 Ludwigshafen (DE)
(72) Erfinder: SÜLING, Carsten, D-51519 Odenthal (DE); GREGORIUS, Heike, D-55543 Bad Kreuznach (DE); DOBLER, Walter, D-69126 Heidelberg (DE); HINGMANN, Roland, D-68526 Ladenburg (DE); RIEGER, Bernhard, D-89075 Ulm (DE); DIETRICH, Ulf, D-89079 Ulm (DE); WAGNER, Jürgen, Matthäus, D-89160 Dornstadt (DE); MÜLLER, Hans Joachim, D-67269 Grünstadt (DE)
(86) Internationale Anmeldenummer: EP9703029
(87) Internationale Veröffentlichungsnummer: WO9749661

(56) Entgegenhaltungen:
- EP-A- 0 545 304
- EP-A- 0 587 107

## Beschreibung

Gegenstand der vorliegenden Erfindung ist ein Verfahren zur Herstellung einer Verbindung der allgemeinen Formel I 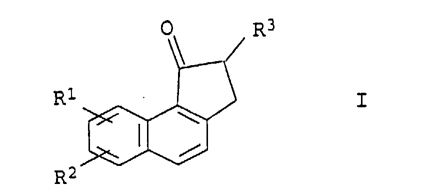 worin R¹ und R² Wasserstoff oder C₁-C₄-Alkyl und R³ einen C₁-C₁₀-Alkylrest, einen Phenylrest oder einen C₁-C₄-alkyl-substituierten Phenylrest bedeuten.

Indanone der allgemeinen Formel I sind wichtige Vorstufen für Metallocenkomplexe, die als Polymerisationskatalysatoren Verwendung finden. Dazu werden die Indanone reduziert und dehydratisiert; die resultierenden Indenderivate können mit Übergangsmetallen zu den entsprechenden Metallocenkomplexen umgesetzt werden.

Zur Herstellung substituierter Indanone sind verschiedene Verfahren bekannt. In der EP-A1-549 900 wird die Synthese eines substituierten Benzindanons beschrieben. Der Fünfring wird dabei in einer mehrstufigen Synthese durch Umsetzung mit einem Malonester, alkalische Verseifung des Diesters, thermische Decarboxylierung, Chlorierung der verbleibenden Carboxylgruppe und intramolekulare Friedel-Crafts-Acylierung gebildet. Die Synthese ist durch ihre Vielstufigkeit mit großem technischen Aufwand verbunden.

In der EP-A1-567 953 wird eine Synthese von Indanonderivaten beschrieben, wobei der Fünfring durch Umsetzung eines entsprechenden Benzolderivates mit einem substituierten Acrylsäureester in flüssigem Fluorwasserstoff gebildet wird. EP-A1-587 107 beschreibt die Herstellung von 2-Methyl-benzindanon durch Umsetzung von Naphthalin mit Methacrylsäureanhydrid in Gegenwart von BF₃/Fluorwasserstoff. Auch diese Synthesewege sind wegen der schwierigen Handhabung der äußerst toxischen Flußsäure mit erheblichem technischen Aufwand verbunden.

Ein weiterer Syntheseweg für Indanone wird in der EP-A1-545 304 beschrieben. Die Herstellung erfolgt hier durch Umsetzung eines Benzolderivates mit α-Halogenalkylpropionsäurehalogeniden, bevorzugt mit α-Bromisobuttersäurebromid. Nachteilig an dieser Synthese ist der große Anfall halogenhaltiger Abfälle.

Der vorliegenden Erfindung lag daher die Aufgabe zugrunde, einen einfachen Syntheseweg zur Herstellung von Benzindanonen der allgemeinen Formel I zu finden, der die Nachteile der bekannten Synthesewege überwindet.

Demgemäß wurde ein Verfahren zur Herstellung einer Verbindung der allgemeinen Formel I 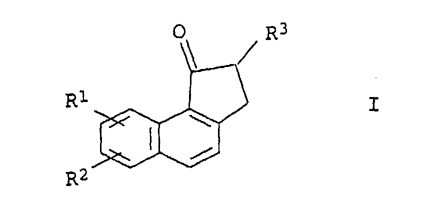 worin R¹ und R² Wasserstoff oder C₁-C₄-Alkyl und R³ einen C₁-C₁₀-Alkylrest, einen Phenylrest oder einen C₁-C₄-alkyl-substituierten Phenylrest bedeuten, gefunden, dadurch gekennzeichnet, daß man eine Verbindung II 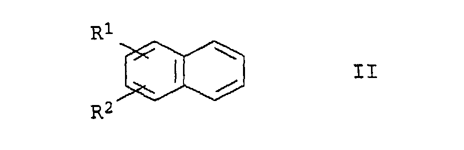 mit einer Verbindung der Formel III 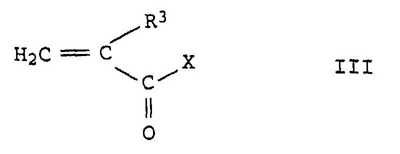 in welcher X Chlor, Brom oder Jod bedeutet, in Gegenwart eines Friedel-Crafts-Katalysators in einem Schritt zu einer Verbindung I umsetzt.

Bei der Umsetzung der Verbindung II mit einer Verbindung III können verschiedene Isomere entstehen. Das erfindungsgemäße Verfahren weist jedoch eine ausgeprägte Regioselektivität auf, so daß meist nur das Isomere I beobachtet wird.

In den Indanonderivaten I, die nach dem erfindungsgemäßen Verfahren herstellbar sind, können die Substituenten R¹ und R² neben Wasserstoff beispielsweise Methyl, Ethyl, Isopropyl, n-Propyl, n-Butyl, Isobutyl, sec-Butyl oder tert-Butyl bedeuten, wobei Wasserstoff besonders bevorzugt ist. Unter den Alkylresten ist besonders der Isopropylrest zu nennen, vorzugsweise ist jedoch nur einer der Reste R¹ oder R² ein Alkylrest.

Der Substituent R³ wird über die Verbindung III in die Zielverbindungen eingeführt. R³ ist vorzugsweise Methyl oder Ethyl, besonders bevorzugt Methyl, jedoch kommen auch andere C₁-C₁₀-Alkylreste, der Phenylrest oder C₁-C₄-alkylsubstituierte Phenylreste in Betracht. Als C₁-C₁₀-Alkylreste kommen neben den obengenannten C₁-C₄-Alkylresten auch die verschiedenen Isomeren Pentyl-, Hexyl-, Heptyl-, Octyl-, Nonyl- und Decylreste in Betracht. Als Phenylreste kommen neben dem bevorzugten unsubstituierten Phenylrest besonders einfach mit Methyl oder Ethyl substituierte Phenylreste in Betracht.

Der Rest X in der Verbindung III kann Chlor, Brom oder Jod bedeuten, bevorzugt ist X Chlor.

Das erfindungsgemäße Verfahren nutzt als Katalysator einen Friedel-Crafts-Katalysator. Als Friedel-Crafts-Katalysatoren kommen alle üblichen derartigen Katalysatoren in Frage, beispielsweise AlCl₃, AlBr₃, BF₃, ZnCl₂, FeCl₃, SnCl₄ und SbCl₅, vorzugsweise wird AlCl₃ verwendet.

Der Friedel-Crafts-Katalysator, insbesondere AlCl₃, wird im allgemeinen im Überschuß eingesetzt, vorzugsweise in einer Menge, daß das Molverhältnis von Katalysator zur Verbindung III zwischen 1 und 2, besonders bevorzugt zwischen 1 und 1,5, ganz besonders bevorzugt zwischen 1,05 und 1,3 beträgt.

Für das erfindungsgemäße Verfahren sind alle üblichen Friedel-Crafts-Lösungsmittel geeignet, wobei Schwefelkohlenstoff, z.B. wegen seiner Toxizität, weniger geeignet ist. In Betracht kommen besonders chlorierte Kohlenwasserstoffe wie Methylenchlorid, 1,2-Dichlorethan, 1,1,2,2-Tetrachlorethan und Chlorbenzol, besonders bevorzugt dient Methylenchlorid als Lösungsmittel.

Die Umsetzung wird bevorzugt bei niedrigen Temperaturen zwischen -30 und 0°C durchgeführt, besonders bevorzugt zwischen -20 und -10°C. Zum Ende der Umsetzung kann zur Vervollständigung der Ringschlußreaktion die Temperatur jedoch angehoben werden, z.B. auf 10-40°C, vorzugsweise auf 20-30°C.

Der Druck hat im allgemeinen keinen nennenswerten Einfluß auf die Reaktion, so daß üblicherweise bei Normaldruck gearbeitet wird.

Das erfindungsgemäße Verfahren zeichnet sich durch einfache technische Handhabung (Einstufenverfahren) und geringen Anfall halogenhaltiger Abfälle aus. Zugleich weist es eine ausgezeichnete Regioselektivität auf und liefert die gewünschten Produkte in sehr guten Ausbeuten.

Das folgende Beispiel erläutert die Erfindung:

### Beispiel

Herstellung von 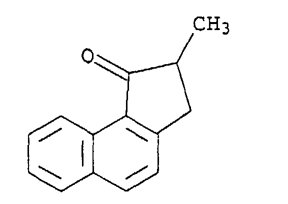 Frisch gepulvertes Aluminiumchlorid (13 g, 97 mmol) wurde in 42 ml CH₂Cl₂ gelöst und auf -15°C abgekühlt. Methacrylsäurechlorid (97 %, 8 ml, 8,6 g, 81 mmol) wurde so langsam zugetropft, daß die Temperatur nicht über -10°C stieg. Anschließend wurde eine Lösung von 10,4 g Naphthalin (81 mmol) in 42 ml CH₂Cl₂ so langsam zugetropft, daß die Temperatur nicht über -10°C stieg. Die Lösung wurde 2 h bei -15°C und 2 h bei Zimmertemperatur gerührt und dann mit 300 ml Eiswasser hydrolysiert. Ausgefallenes Aluminiumhydroxidgel wurde mit etwas HCl in Lösung gebracht, und die organische Phase wurde abgetrennt. Die wäßrige Phase wurde noch dreimal mit 80 ml CH₂Cl₂ extrahiert, die vereinigten organischen Phasen wurden mit gesättigter NaHCO₃-Lösung und dann mit gesättigter NaCl-Lösung gewaschen und über Na₂SO₄ getrocknet.

Nach Verdampfen des CH₂Cl₂ wurde der Rückstand in Ethylacetat aufgenommen und durch Filtration über eine kurze Säule Kieselgel gereinigt. Erneutes Verdampfen des Lösemittel ergab ein rötliches zähes Öl, das langsam fest wurde. Das ¹H-NMR-Spektrum entsprach den Literaturdaten (EP 0 545 304).

Die Bildung des isomeren 2-Methyl-4,5-benzoindan-3-ons, das bei der Synthese in EP 0 545 304 ebenfalls entstand, sowie die Bildung von 2-Methyl-5,6-benzoindan-1-on wurde nicht beobachtet.
Ausbeute: 15,1 g

## Patentansprüche

1. Verfahren zur Herstellung einer Verbindung der allgemeinen Formel I 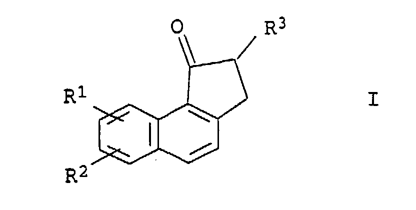 worin R¹ und R² Wasserstoff oder C₁-C₄-Alkyl und R³ einen C₁-C₁₀-Alkylrest, einen Phenylrest oder einen C₁-C₄-alkyl-substituierten Phenylrest bedeuten, dadurch gekennzeichnet, daß man eine Verbindung II 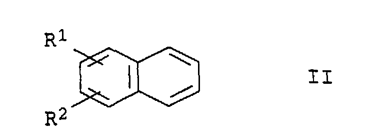 mit einer Verbindung der Formel III 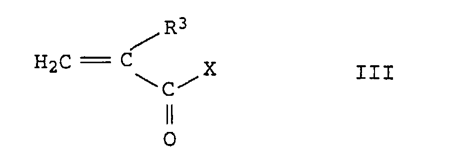 in welcher X Chlor, Brom oder Jod bedeutet, in Gegenwart eines Friedel-Crafts-Katalysators in einem Schritt zu einer Verbindung I umsetzt.

2. Verfahren nach Anspruch 1, dadurch gekennzeichnet, daß R¹ und R² Wasserstoff bedeuten.

3. Verfahren nach den Ansprüchen 1 und 2, dadurch gekennzeichnet, daß R³ Methyl oder Ethyl bedeutet.

4. Verfahren nach den Ansprüchen 1 bis 3, dadurch gekennzeichnet, daß als Friedel-Crafts-Katalysator AlCl₃ eingesetzt wird.

5. Verfahren nach den Ansprüchen 1 bis 4, dadurch gekennzeichnet, daß das Molverhältnis von AlCl₃ zur Verbindung III 1,0 bis 1,5 beträgt.

6. Verfahren nach den Ansprüchen 1 bis 5, dadurch gekennzeichnet, daß die Umsetzung in Gegenwart von Methylenchlorid durchgeführt wird.

7. Verfahren nach den Ansprüchen 1 bis 6, dadurch gekennzeichnet, daß die Umsetzung bei Temperaturen zwischen -30 und 0°C durchgeführt wird.

## Claims

1. A process for preparing a compound of the formula I 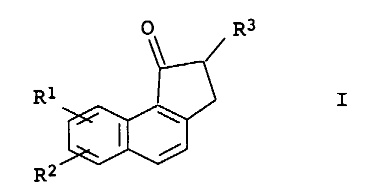 where R¹ and R² are hydrogen or C₁-C₄-alkyl and R³ is C₁-C₁₀-alkyl, phenyl or C₁-C₄-alkyl-substituted phenyl, which comprises reacting a compound II 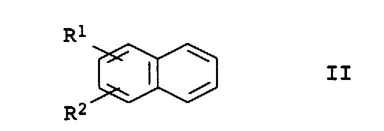 with a compound of the formula III 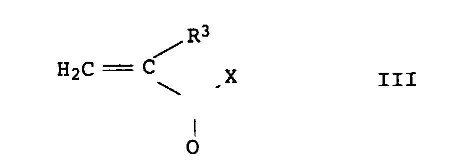 where X is chlorine, bromine or iodine, in the presence of a Friedel-Crafts catalyst in one step to give a compound I.

2. A process as claimed in claim 1, wherein R¹ and R² are hydrogen.

3. A process as claimed in either of claims 1 and 2, wherein R³ is methyl or ethyl.

4. A process as claimed in any of claims 1 to 3, wherein AlCl₃ is employed as Friedel-Crafts catalyst.

5. A process as claimed in any of claims 1 to 4, wherein the molar ratio of AlCl₃ to compound III is from 1.0 to 1.5.

6. A process as claimed in any of claims 1 to 5, wherein the reaction is carried out in the presence of methylene chloride.

7. A process as claimed in any of claims 1 to 6, wherein the reaction is carried out at from -30 to 0°C.

## Revendications

1. Procédé de préparation d'un composé de formule générale I 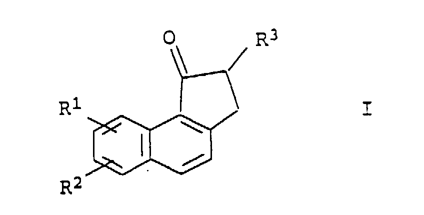 dans laquelle R¹ et R² représentent l'hydrogène ou des groupes alkyle en C1-C4 et R³ un groupe alkyle en C1-C10, un groupe phényle ou un groupe phényle à substituants alkyle en C1-C4, caractérisé par le fait que l'on fait réagir un composé II 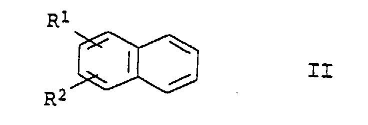 avec un composé de formule III 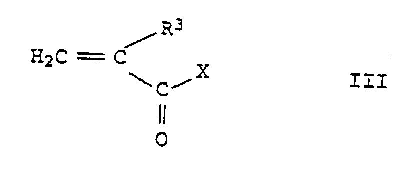 dans laquelle X représente le chlore, le brome ou l'iode, en présence d'un catalyseur de Friedel-Crafts, en un seul stade opératoire, la réaction donnant un composé I.

2. Procédé selon la revendication 1, caractérisé par le fait que R¹ et R² représentent l'hydrogène.

3. Procédé selon les revendications 1 et 2, caractérisé par le fait que R³ représente un groupe méthyle ou éthyle.

4. Procédé selon les revendications 1 à 3, caractérisé par le fait que le catalyseur de Friedel-Crafts mis en oeuvre est AlCl₃.

5. Procédé selon les revendications 1 à 4, caractérisé par le fait que le rapport molaire entre AlCl₃ et le composé III va de 1,0 à 1,5.

6. Procédé selon les revendications 1 à 5, caractérisé par le fait que la réaction est effectuée en présence de chlorure de méthylène.

7. Procédé selon les revendications 1 à 6, caractérisé par le fait que la réaction est effectuée à des températures allant de -30 à 0°C.
